# EUROPEAN PATENT APPLICATION

(11) **EP 1 923 060 A1**
(43) Date of publication of application: **21.05.2008**
(21) Application number: 06076993.2
(22) Date of filing: 08.11.2006
(51) Int. Cl.: A61K 31/133, A61K 31/40, A61K 38/00, A61P 3/00, A61P 3/06, A61P 43/00

(54) **Combinations of a sphingolipid and an HMG-CoA reductase inhibitor for treating hypercholesterolemia**

(71) Applicant: Nederlandse Organisatie voor Toegepast-Natuurwetenschappelijk Onderzoek TNO, 2628 VK Delft (NL)
(72) Inventor: Nieuwenhuizen, Willem Ferdinand, 3981 AM Bunnik (NL); Emeis, Josephus Jan, 1018 BE Amsterdam (NL); Havekes, Aloysius Maria, 2402 PP Alphen aan den Rijn (NL); Kats, Mark Paul, 3971 PT Driebergen-Rijsenburg (NL)
(74) Representative: van Loon, C.J.J.

(57) **Abstract**

The present invention relates to the use of a sphingolipid and a cholesterol lowering agent, wherein said cholesterol lowering agent is a HMG-CoA reductase inhibitor, for the manufacture of a combined preparation for simultaneous, separate or sequential use in the treatment of hypercholesterolemia in a subject in need thereof.

## Description

### TECHNICAL FIELD

The invention relates to preparations for the treatment and prevention of hypercholesterolemia. In particular, the present invention relates to the use of sphingolipids, more preferably phytosphingosine, sphingosine, sphinganine, ceramide, glycosylceramide and/or sphingomyelin and statins for the manufacture of a combined preparation for simultaneous, separate or sequential use in the treatment of hypercholesterolemia in a subject in need thereof.

### BACKGROUND OF THE INVENTION

Statins belong to the most widely prescribed preventive medicaments used for the prophylaxis of cardiovascular disease, with tens of millions of users world-wide. Statins, also known as HMG-CoA reductase inhibitors, are hypolipidemic agents used primarily for lowering low density lipoprotein (LDL) cholesterol and triglyceride levels. The drugs block endogenous cholesterol synthesis in the liver by inhibiting HMG-CoA reductase in the early steps of the mevalonate synthesis pathway. Ultimately, often in combination with a low-cholesterol diet to reduce exogenous uptake from the intestine, this results in lower blood cholesterol levels.

Examples of presently available statins are atorvastatin, cerivastatin, fluvastatin, lovastatin, mevastatin, pitavastatin, pravastatin, rosuvastatin, and simvastatin.

Although statins are considered very valuable medicaments in the prophylaxis and treatment of cardiovascular disease, the long term use of statins appears to be associated with damage to the liver as a side effect. Yet, there are at present no suitable alternatives to statins.

It is an aim of the present invention to provide additional preparations for the treatment and prevention of hypercholesterolemia.

It is another aim of the present invention to provide more effective cholesterol-lowering compositions.

It is another aim of the invention to provide preparations that will allow for effective treatment or prophylaxis of hypercholesterolemia in accordance with treatment regimens that require lower dosages of statins, or alternatively, to provide for a higher reduction in blood cholesterol levels at equal dosage levels of statins.

### SUMMARY OF THE INVENTION

In relation thereto, the present inventors have now found that a combined preparation of sphingolipids and HMG-CoA reductase inhibitors may very suitably be used for the treatment of hypercholesterolemia. As a result, the medicaments comprising sphingolipids and HMG-CoA reductase inhibitors may suitably be used to prevent the occurrence of the more damaging consequences of the HMG-CoA reductase inhibitors on the liver. Due to this capacity, the medicaments comprising sphingolipids and HMG-CoA reductase inhibitors may be used in accordance with the present invention for the highly effective prevention and/or treatment of hypercholesterolemia.

In one aspect, the invention now provides the use of a sphingolipid and a cholesterol lowering agent, wherein said cholesterol lowering agent is a HMG-CoA reductase inhibitor, for the manufacture of a combined preparation for simultaneous, separate or sequential use in the treatment of hypercholesterolemia in a subject in need thereof. In a particularly preferred embodiment, said treatment of hypercholesterolemia involves the simultaneous treatment or prophylaxis of liver damage.

In another preferred embodiment of said use, the cholesterol lowering agent is administered to said subject prior to or following the administration of the sphingolipid.

In another preferred embodiment of said use, the sphingolipid has the general formula (I): 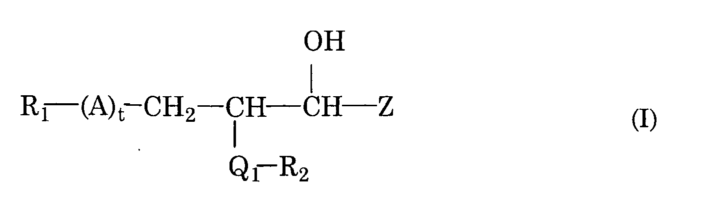 wherein
Z is R₃ or -CH(OH)-R₃;
A is sulphate, sulphonate, phosphate, phosphonate or -C(O)O-;
R₁ is H, hydroxyl, alditol, glycosyl, an alcohol, C₁-C₆ alkyl or amino acid;
R₂ is H or unsaturated or saturated (C₁-C₃₀) alkyl chain;
R₃ is unsaturated or saturated (C₁-C₃₀) alkyl chain;
Q₁ is a primary amine group (-NH₂), secondary amine group (-NH-) or an amide group (-NH-CO-).

In another preferred embodiment of said use, the sphingolipid has the general formula (II) 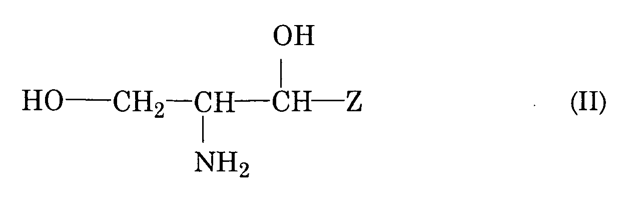 wherein
Z is R₃ or CH(OH)-R₃, and
R₃ is an unsaturated or saturated (C₁-C₃₀) alkyl chain.

In another preferred embodiment of said use, the sphingolipid has the general formula (III) 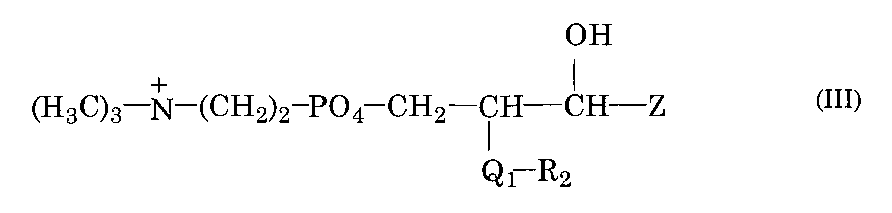 wherein
Z is R₃ or CH(OH)-R₃, preferably R₃;
Q₁ is a primary amine group (-NH₂), a secondary amine group (-NH-) or an amide group (-NH-CO-); preferably an amide group, and
R₂ is H or unsaturated or saturated (C₁-C₃₀) alkyl chain;
R₃ is an unsaturated or saturated (C₁-C₃₀) alkyl chain, preferably an
unsaturated (C₁-C₃₀) alkyl chain.

In another preferred embodiment of said use, the sphingolipid has the general formula (IV), 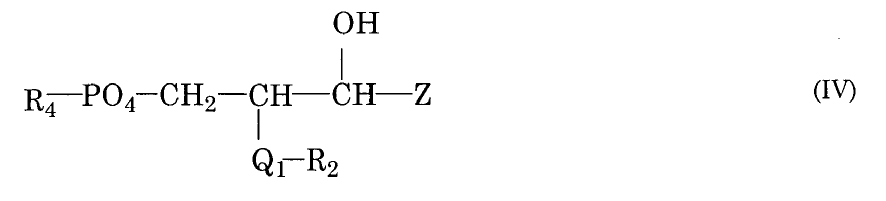 wherein
Z, Q₁, and R₂ are as defined above, and
R₄ is selected from H, hydroxyl, alditol, glycosyl, alcohol, C₁-C₆ alkyl or amino acid, provided that when R₄ is H, Q1-R₂ is not the primary amine group. The hydroxyl, alditol, alcohol, C₁-C₆ alkyl or amino acid can for instance be those compounds as named for R₁ above. In case R₄ is glycosyl, it may be selected from the group of radicals consisting of acesulfam, allose, altrose, arabinose, erythrose, fructose, fucose, galactose, glucose, gulose, idose, isomaltose, lactose, lyxose, maltose, mannose, melezitose, psicose, raffinose, rhamnose, ribose, saccharose, sorbose, stachyose, sucrose, tagatose, talose, threose, trehalose, turanose, xylose and xylulose, and other mono-, di-, or polysaccharides.

In another preferred embodiment of said use, the sphingolipid is selected from the group consisting of phytosphingosine, sphingosine, sphinganine, ceramide, glycosylceramide, sphingomyelin, and derivatives thereof, and combinations thereof, preferably said sphingolipid is phytosphingosine.

In another preferred embodiment of said use, the cholesterol lowering agent is a statin, preferably a statin selected from the group consisting of atorvastatin, cerivastatin, fluvastatin, lovastatin, mevastatin, pitavastatin, pravastatin, rosuvastatin, and simvastatin.

In another preferred embodiment of said use, the daily dose of the sphingolipid is between about 0.1 and about 50 mg/kg body weight.

In another preferred embodiment of said use, the statin is administered at a dose of about 0.001 to about 5 mg/kg body weight.

In another aspect, the invention provides a combined preparation of a sphingolipid and at least one cholesterol lowering agent, wherein said cholesterol lowering agent is a HMG-CoA reductase inhibitor.

In another aspect, the invention provides a combined preparation of a sphingolipid and at least one cholesterol lowering agent, wherein said cholesterol lowering agent is a HMG-CoA reductase inhibitor, preferably a statin, for the simultaneous, separate or sequential use in therapy.

In a preferred embodiment, the sphingolipid is selected from the group consisting of phytosphingosine, sphingosine, sphinganine, ceramide, glycosylceramide, sphingomyelin, and derivatives thereof, and combinations thereof.

In still another aspect, the invention provides a method of treating hypercholesterolemia in a subject, said method comprising administering to said subject a therapeutically effective amount of a preparation according to the invention.

The medicaments, pharmaceutical compositions and preparations of the present invention are preferably for oral administration.

### DESCRIPTION OF THE DRAWINGS

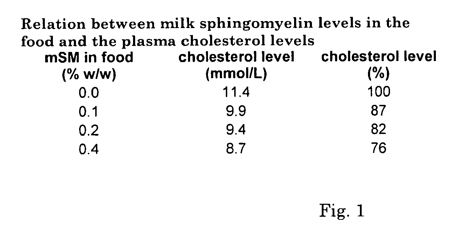

Figure 1 shows in a table the relation between milk sphingomyelin levels in the food and the plasma cholesterol levels as found in a pilot study. Data are used to determine effective dosage levels in the Example.

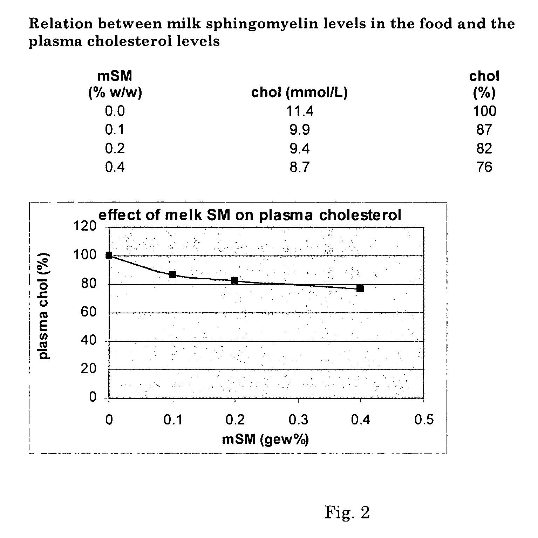

Figure 2 shows data (in table and graphic display) from a pilot study wherein the effect of milk sphingolipids on plasma cholesterol levels in hypercholesterolemic APOE*3Leiden mice was investigated. Data are used to determine effective dosage levels in the Example.

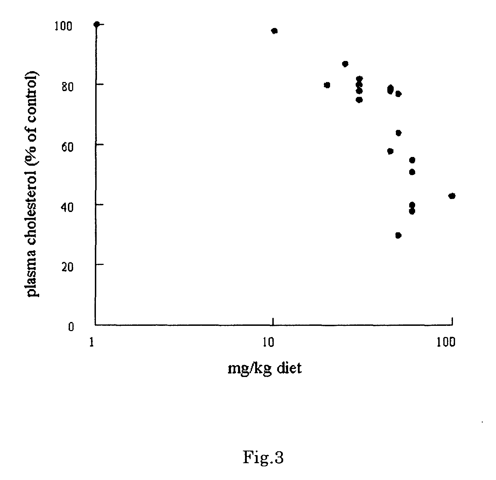

Figure 3 shows the relationship between Atorvastatin concentration in the diet (in mg Atorvastatin per kg diet) and the reduction of plasma cholesterol after two weeks in APOE*3Leiden mice on a hypercholesterolemic diet. Data are used to determine effective dosage levels in the Example.

### DEFINITIONS

The term "statin" and its plural form "statins", is used herein to refer to a group of HMG-CoA reductase inhibitors. Currently available statins include atorvastatin (Lipitor, Torvast), cerivastatin (Lipobay, Baycol), fluvastatin (Lescol), lovastatin (Mevacor, Altocor), mevastatin, pitavastatin (Livalo, Pitava), pravastatin (Pravachol, Selektine, Lipostat), rosuvastatin (Crestor), and simvastatin (Zocor, Lipex). Lovastatin (disclosed in U.S. Pat. No. 4,231,938) and simvastatin (disclosed in U.S. Pat. No. 4,444,784 and WO 00/53566) are usually administered in the lactone form. After absorption, the lactone ring is opened in the liver by chemical or enzymatic hydrolysis, and the active hydroxy acid is generated. Pravastatin (disclosed in U.S. Pat. No. 4,346,227) is usually administered as the sodium salt. Fluvastatin (disclosed in U.S. Pat. No. 4,739,073) and cerivastatin (disclosed in U.S. Pat. Nos. 5,006,530 and 5,177,080), also usually administered as the sodium salt, are entirely synthetic compounds that are in part structurally distinct from the fungal derivatives of this class that contain a hexahydronaphthalene ring. Atorvastatin (U.S. Pat. Nos. 4,681,893, 5,273,995, 5,273,995, and 5,298,627), rosuvastatin (U.S. Pat. No. 5,260,440) and pitavastatin (U.S. Pat. Nos. 5,011,930, 5,856,336 and 5,872,130) are usually administered as calcium salts. Processes for preparing calcium salt forms of statins are disclosed in US2005197501.

As used herein, the term "steatosis" refers to the condition in which fat accumulates in tissues, such as liver tissue, heart muscle tissue or other muscle tissues. The term "steatosis" does not imply any causative relationship with any metabolic condition or disorder.

As stated earlier, the term "hepatic steatosis" refers to the condition in which fat accumulates in the liver. As used herein, the term "hepatic steatosis" does not imply any causative relationship with any metabolic condition or disorder.

As used herein, the term "hepatic fibrosis" is used in its art-recognised meaning and refers to the clinically silent 'wound healing' process initiated in response to continuous insult to hepatic tissue.

As used herein, the term "hepatic cirrhosis" is used in its art-recognised meaning and refers to a group of chronic diseases of the liver in which normal liver cells are damaged and replaced by scar tissue, decreasing the amount of normal liver tissue.

As used herein, the term "sphingolipid" refers to a member of a class of lipids derived from the aliphatic amino alcohol sphingosine. The sphingosine backbone is O-linked to a (usually) charged head group such as ethanolamine, serine, or choline. Bonding may occur via a phosphodiester linkage. The backbone is also amide-linked to an acyl group such as a fatty acid. Sphingolipids are often found in neural tissue, and play an important role in both signal transmission and cell-cell recognition. There are three main types of sphingolipids: ceramides, sphingomyelins, and glycosphingolipids. The term is specifically used to address the group of compounds according to the formulas (I), (II), (III) and (IV) of the present invention, including analogs or derivatives or pharmaceutically acceptable salts thereof, alone, or in combination, or as a so-called precursor compound, unless explicitly noted otherwise. The sphingolipids used in aspects of the present invention are used in non-complexed, non-bound form, i.e. in free form, not covalently linked to another (bio)molecule.

The term "therapeutically effective amount" as used herein refers to an amount of a therapeutic agent to treat, ameliorate, or prevent a disease or condition, or to exhibit a detectable therapeutic or prophylactic effect. The precise effective amount needed for a subject will depend upon the subject's size and health, the nature and extent of the condition, and the therapeutics or combination of therapeutics selected for administration. Thus, it is not useful to specify an exact effective amount in advance. However, the effective amount for a given situation can be determined by routine experimentation.

A "derivative", "analog" or "analogue" is defined herein as a sphingolipid according to the formula (I), (II), (III) or (IV) that is subjected to a (bio)chemical modification (e.g. organo-chemical or enzymatical). Derivatising may comprise the substitution of certain chemical groups to the sphingolipid, thereby retaining the sphingolipid character of the compound. Such derivatizations are known in the art. The derivatives and analogues maintain the biological activity of the natural sphingolipid i.e. in that they exhibit an hepatoprotective effect, but may provide advantages to the molecule such as longer half-life, resistance to degradation or an increased activity. A very suitable derivative of phytosphingosine is for instance tetra acetyl phytosphingosine (TAPS, see below). Such a derivative may suitably be used in embodiments of the present invention since after hydrolysis, for instance in the body, the converted compound will exert its liver healing effects.

A "pharmaceutically acceptable salt" is defined herein as a salt wherein the desired biological activity of the sphingolipid is maintained and which exhibits a minimum of undesired toxicological effects. Non-limiting examples of such a salt are (a) acid addition salts formed with inorganic acids (e.g., hydrochloric acid, hydrobromic acid, sulphuric acid, phosphoric acid, nitric acid, and the like), and salts formed with organic acids (such as e.g. acetic acid, oxalic acid, tartaric acid, succinic acid, malic acid, ascorbic acid, benzoic acid, tannic acid, palmitic acid, polyglutamic acid, naphthalene sulphonic acid, naphthalene disulphonic acid, polygalacturonic acid and the like); (b) base addition salts formed with metal cations such as zinc, calcium, bismuth, barium, magnesium, aluminium, copper, cobalt, nickel, cadmium, sodium, potassium and the like, or with a cation formed from ammonia, N,N-dibenzylethylene-diamine, D-glucosamine, tetraethylammonium or ethylenediamine; or (c) combinations of (a) and (b); e.g. a zinc tannate or the like. The use of a pharmaceutically acceptable salt of a sphingolipid according to the formula (I), (II), (III) or (IV), such as an ammonium salt or a chloride salt is preferred since the salt form is better soluble and will thus enhance the bio-availability of the sphingolipid. Preferably a salt of HCl is used. The use of a pharmaceutically acceptable salt is not limited to pharmaceutical preparations, but includes the use in food items or food supplements.

A "precursor" is defined herein as a derivative of the active compound with similar, less or no activity, and which can be transformed to the active compound e.g. by the digestive tract or other digestive systems in the body. Such precursors can be obtained by chemical or enzymatic modification of the active molecule.

"Subject" as used herein includes, but is not limited to, mammals, including, e.g., a human, non-human primate, mouse, pig, cow, goat, cat, rabbit, rat, guinea pig, hamster, horse, monkey, sheep, or other non-human mammal; and non-mammal animals, including, e.g., a non-mammalian vertebrate, such as a bird (e.g., a chicken or duck) or a fish, and an invertebrate.

### DETAILED DESCRIPTION OF THE INVENTION

The present invention is based on the insight that sphingolipids and statins have a synergistic effect in lowering plasma cholesterol levels. A synergistic effect is defined as an interaction of two or more drugs such that their combined effect is greater than the sum of the individual effects seen when each drug is given alone or the potentiation of one drug by one or more additional agents when administered together.

An important advantage of the present invention is further that the liver damage that may result from statin is at the same time counteracted by the hepatoprotective activity of the sphingolipid. Moreover, due to the additional cholesterol-lowering effect of the sphingomyelin, a desired reduction in the blood cholesterol may be obtained by using lower amounts of statins, which would also effectively reduce the changes for liver damage.

Such liver damage may occur in up to 1-3% of regular users. Especially in elderly patients and in patient with frequent alcohol consumption, statin use may pose a risk factor for developing liver damage. In order to demonstrate such liver damage, markers are available. For instance, it is known that high levels in blood of the liver enzyme Alanine Amino Transferase (abbreviated as ALT in human and ALAT in mice) and Serum Amiloid A (SAA), are indicators for the onset of liver damage. In fact, the US Food and Drug Administration will refuse approval of a statin if the rise in blood level of these markers is too strong in clinical trials. Moreover, liver parameters such as ALT are regularly monitored in patients

If liver damage, or the onset thereof, is diagnosed in patients taking statins, there is at present no other solution than to switch medication to a different type of statin, or to revert to another, less effective, therapy.

Therefore, there is at present a need for medicaments for the treatment of hypercholesterolemia, while preventing and/or treating the hepatotoxic effects of HMG-CoA reductase inhibitors, and to prevent the more damaging consequences thereof.

Sphingolipids have been associated with a number of beneficial effects, however, it has now also been found that sphingolipids can effectively prevent liver damage associated with statin use.

Sphingolipids are lipids of which some occur in food in low concentrations and which form a minor but important constituent of the cells of plants, animals and man. Since several sphingolipids occur naturally in the body of man and animal, they will be easily acceptable as excipients or adjuvant in pharmaceutical agents.

Sphingolipids are generally composed of a long sphingoid base (sphingosine, sphinganine, phytosphingosine, or a related compound) as the central group of the molecule or "backbone" (see *intra alia* Karlsson. 1970. Chem. Phys. Lipids, 5:6-43), which may comprise an amide-linked long-chain fatty acid and a head group. There are hundreds of different molecular species of sphingolipids with different head groups (e.g. cholinephosphate, glucose, galactose, polysaccharides) and with different fatty acids and sphingoid bases (see *intra alia* Merrill & Sweeley. 1996. New Comprehensive Biochemistry: Biochemistry of Lipids, Lipoproteins, and Membranes, (Vance, D.E. & Vance, J.E., eds.), pp. 309-338, Elsevier Science, Amsterdam).

The simplest sphingolipids, like sphingosine and sphinganine normally occur in food in very low concentrations. The richest sources of sphingolipids are dairy products, soy beans, eggs, meat, including fish meat, shellfish meat and meat of marine invertebrates, such as starfish. The most abundant sphingolipids in food are sphingomyelin (milk and eggs) and ceramide (meat). Whole milk contains predominantly sphingomyelin, but also contains glucosylceramide, lactosylceramide and gangliosides. Potato, apple, tomato, spinach, pepper and rice especially contain glycosylceramides in low concentration (see, e.g. Stryer L. 1988. Biochemistry, p. 287 [W.H. Freeman and Co., NY, USA]; Ryu J. et al. 2003. Arch Pharm Res. Feb;26(2):138-42; Kawatake S, et al. 2002. Chem Pharm Bull (Tokyo) 50(8):1091-6).

It is known that sphingosine and sphingosine-analogs inhibit growth and metastasis of human and animal tumor cells (see e.g. EP 0 381 514). It is also known that administration of sphingomyelin to the food of rats can significantly decrease the chances of occurrence of malignant, chemically induced colon cancer.

Sphingolipids are also used in pharmaceutical compositions to protect skin and/or hair against the damaging effects of air pollution (see e.g. US 5.869.034).

The antimicrobial action of sphingosine as a component of the skin against bacteria such as *Staphylococcus aureus, Candida albicans* and *Propionibacterium acnes* is known from dermatology (Bibel et al. 1992. J. Invest. Dermatol. 98(3):269-73; Bibel et al. 1995. Clin Exp Dermatol 20(5):395-400), and the application of topical ointments comprising sphingosine is described therein.

WO 00/50574 discloses the use of anti-lipemic drugs comprising an effector of SREBP-1 associated with (linked to) a known serum cholesterol inhibitor such as fluvastatin, simvastatin, lovastatin for modulating serum cholesterol. Sphingomyelin and ceramide are disclosed as suitable effectors. However, an antisteatotic effect of sphingomyelin or ceramide in free form is not described.

US 2002/0182250 describes a lipid metabolism improving agent comprising a protein/phospholipid complex wherein the phospholipids is bound to the protein(hydrolysate) for use in the treatment or prevention of a variety of diseases including fatty liver. Sphingomyelin is mentioned amongst the phospholipids. However, it is not disclosed which of the ingredients of the agent is responsible for any of the claimed effects, and an antisteatotic effect of the free phospholipids is not described.

US 2004/0171557 describes that liver fat content in C57bI mice may be reduced by intraperitoneal injection of glucocerebroside. The oral administration of sphingolipids is however not disclosed herein.

WO 03/011873 discloses the use of a phospholipids-comprising extract of marine or aquatic crustaceans or zooplankton for treating a wide variety of disorders. Among the disorders presented are liver disease, steatosis and liver fibrosis. However, it is not disclosed in this prior art citation which of the many different ingredients of the extract is responsible for any of the claimed effects.

EP 1 452 181 describes the applicability of intermediary metabolites, such as glucosylceramides to threat various diseases. No reference is made to diseases of the liver.

Kim et al. (Phytotherapy Res. (2000) 14:448-451) describe the cerebroside LCC (1-*O*-(β-D-glucopyranosyl)-(2*S*,3*R*,4*E*,8*Z*)-2-*N-*palmityloctadecasphinga-4,8-diene) isolated from *Lycium chinense* as a potential hepatoprotective agent. Basis for this assumption is the observed ability of the compound to reduce the release into the culture medium of GPT and SDH from galactosamine-injured primary rat hepatocytes. No disclosure is made of a suitable use in the treatment and prevention of steatosis, i.e. of excessive fat accumulation in the liver of the whole organism.

The present inventors have now found that sphingolipids can be used effectively to prevent hepatotoxic effects of cholesterol lowering agents of the class of HMG CoA reductase inhibitors, such as statins. It is not essential that the statin and sphingolipid are administered together. Rather, they may be administered sequentially or separately. However when the combined preparation is administered simultaneously, the active substances are preferably present in the preparation in the form of an admixture, wherein both the statin and the sphingolipid are in their free (e.g. salt or lactone) form.

The present invention provides the use of a sphingolipid according to the formula (I) 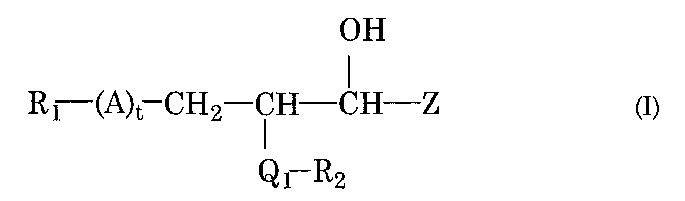 wherein
Z is R₃ or -CH(OH)-R₃;
A is sulphate, sulphonate, phosphate, phosphonate or -C(O)O-;
R₁ is H, hydroxyl, alditol, glycosyl, an alcohol, C₁-C₆ alkyl or amino acid;
R₂ is H or unsaturated or saturated (C₁-C₃₀) alkyl chain;
R₃ is unsaturated or saturated (C₁-C₃₀) alkyl chain;
Q₁ is a primary amine group (-NH₂), secondary amine group (-NH-) or an
amide group (-NH-CO-); preferably an secondary amine group; and t is 0 or 1,
or a precursor, a derivative or a pharmaceutically acceptable salt thereof for the manufacture of a medicament for the prevention and/or treatment of hepatic steatosis, fibrosis and cirrhosis.

R₁ can be selected from glycosyl radicals, preferably R₁ is selected from the group of radicals consisting of acesulfam, allose, altrose, arabinose, erythrose, fructose, fucose, galactose, gulose, idose, isomaltose, lactose, lyxose, maltose, mannose, melezitose, psicose, raffinose, rhamnose, ribose, saccharose, sorbose, stachyose, sucrose, tagatose, talose, threose, trehalose, turanose, xylose and xylulose.

R₁ is preferably selected from amino acids radicals, such as radicals of alanine, arginine, asparagines, aspartate, carnitine, citrulline, cysteine, cystine, GABA, glutamate, glutamine, gluthathione, glycine, histidine, hydroxyproline, isoleucine, leucine, lysine, methionine, ornithine, phenylalanine, proline, serine, taurine, threonine, tryptophane, tyrosine and valine or derivatives or combinations thereof.

R₁ is more preferably selected from the group consisting of hydrogen, hydroxyl or hydroxyl-containing group (e.g. hydroxyalkyl), alditol radical or polyol radical, such as radicals of adonitol, arabitol, dulcitol, erythritol, ethyleneglycol, glycerol, inositol, lactitol, maltitol, mannitol, propyleneglycol, ribitol, sorbitol, threitol and xylitol, and of methanol, ethanol, ethanediol, isopropanol, n-propanol, 1,3-propanediol, and other poly-alcohols.

Even more preferably R₁ is selected from the group consisting of radicals of alcohols such as, choline, ethanolamine, ethanol, glycerol, inositol, tyrosine and serine and still more preferably from the alcohol moieties of phosphoglycerides or phosphoglyceride-alcohols, such as choline, serine, ethanolamine, glycerol or inositol.

R₁ is most preferably a hydroxyl group.

(A) can have any desired counter-ion for the formation of a salt of a sphingolipid according to the formula (I).

It is possible that the amino group such as may be present in the form of Q₁ in a sphingolipid according to the formula (I) is modified, e.g. by single or multiple methylation, alkylation, acylation of acetylation or by modification to a formic acid amide.

Also the free hydroxyl groups in the formula (I), specifically those in R₃ may be modified in ways known to the skilled person.

Further, all possible racemates and (dia)stereoisomers of a sphingolipid according to the formula (I) can be used in the present invention. It is possible to use compounds according to the formula (I) wherein Q₁ is substituted by e.g. H, a hydroxyl, a carboxyl or a cyano group. Preferred is a compound wherein Q₁ is the amino group.

R₂ is H or unsaturated or saturated (C₁-C₃₀) alkyl chain and R₃ is unsaturated or saturated (C₁-C₃₀) alkyl chain.

The term alkyl as used herein refers to a saturated or unsaturated straight chain, branched or cyclic, primary, secondary or tertiary hydrocarbon of C₁- C₃₀, optionally substituted, and comprises specifically methyl, ethyl, propyl, butyl, isobutyl, t-butyl, pentyl, cyclopentyl, isopentyl, neopentyl, hexyl, isohexyl, cyclohexyl, cyclohexylmethyl, 3-methylpentyl, 2,2-dimethylbutyl and 2,3-dimethylbutyl, heptyl, octyl, nonyl, decyl, undecyl, dodecyl, tridecyl, tetradecyl, pentadecyl, hexadecyl, heptadecyl, octadecyl, nonadecyl, eikosyl, heneikosyl and dokosyl and isomers thereof.

The C₁- C₃₀ alkyl chain or -group may be optionally substituted with one or more groups selected from the collection consisting of hydroxyl, amino, alkylamino, arylamino, alkoxy, aryloxy, nitro, cyano, sulphonic acid, sulphate, sulphonate, phosphonate or phosphate, either unprotected or protected insofar as desired. These substitutes are known to the person skilled in the art, for example from Greene et al., Protective Groups in Organic Synthesis, John Wiley & Sons, 2nd Edition, 1991. Preferred embodiments of C₁- C₃₀ alkyl chains constitute C₈-C₂₄ alkyl chains.

A compound of the formula (I) is a sphingolipid, or a precursor, a derivative or pharmaceutically acceptable salt thereof.

Even more preferably, in a compound according to the formula (I), or a precursor, a derivative or a pharmaceutically acceptable salt thereof, R₁ is a hydroxyl group, t is 0, R₂ is hydrogen, R₃ is unsaturated or saturated (C₁-C₃₀) alkyl, Q₁-R₂ together is an amine group. More preferably therefore, a sphingolipid used in embodiments of the present invention is a sphingolipid with the general formula (II): 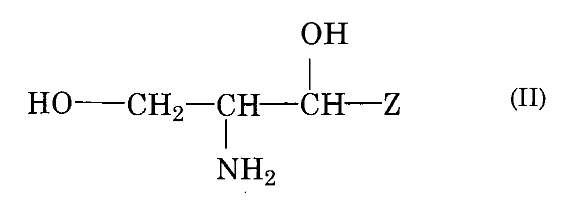 wherein and Z is R₃ or CH(OH)-R₃ and R₃ is an unsaturated or saturated (C₁-C₃₀)alkyl chain.

In an even more preferred embodiment of the present invention, a phytosphingosine, sphingosine, sphinganine, ceramide, glycosylceramide and/or sphingomyelin is used, since these compounds show excellent prevention of hepatic injury. In a most preferred embodiment of the present invention, a phytosphingosine is used.

Because the simple sphingolipids, i.e. the sphingoid bases, such as e.g. phytosphingosine, sphingosine, and sphinganine, have the same effect as more complex sphingolipids such as ceramide III, sphingomyelin and glycosylceramide, it is preferred to use the simple sphingolipids in aspects of the present invention.

Besides sphingomyelin, phytosphingosine, sphingosine, sphinganine, ceramide and glycosylceramide also derivatives of these compounds may be used in aspects of the present invention. For instance, instead of a hydroxyl headgroup, a choline phosphate, ethanolamine phosphate, serine phosphate, inositol phosphate, glycerol phosphate, glucose or galactose head group may be used as R₁ group in a compound according to the formula (I). Basically all headgroups within the definition of R1 above may be used for derivatization of phytosphingosine, sphingosine and sphinganine. A derivative such as lyso-sphingomyelin may also be used in embodiments of the present invention.

It is also possible to use a combination of sphingolipids according to the formula (I), (II), (III) and/or IV in aspects of the present invention.

In principle, sphingolipids according to the formula (I), (II), (III) and/or IV of all possible sources are suitable for use in aspects and embodiments of the present invention. For instance, a suitable sphingolipid such as phytosphingosine may be obtained from plants such as corn (Wright et al., Arch. Biochem. Biophys. 415(2), 184-192 and references therein), from animals (skin fibroblasts) or from micro-organisms such as yeasts (such as *Pichia ciferii*)*.* The sphingolipids may be isolated from these organisms or can be used in a less pure form, i.e. as an enriched fraction, or in the case of microorganisms such as yeasts by taking the complete organism(s) or fractions thereof. Further, sphingolipids may be isolated from other suitable sources, such as from milk, egg, soy, yeast, bacteria, algae, plants, meat, brain, etc. or may be chemically or enzymatically prepared, for use in a pharmaceutical composition according to the invention. Bacterial sources of sphingolipids are e.g. known from US 6,204,006.

Sphingolipids may be derived from the above sources by methods known to the skilled person for instance by extraction with (organic) solvents, chromatographic separation, precipitation, crystallization and/or enzymatic of chemical hydrolysis. The production of a sphingolipid-enriched (specifically a sphingomyelin-enriched) fraction from milk is for instance known from WO94/18289. Sphingolipids may also be derived from fat concentrates of various animal products such as milk products, egg products and blood products such as known from US 5,677,472.

Methods for the preparation of sphingolipids and sphingolipid derivatives are *i.a.* known from EP 0 940 409, WO 98/03529, WO 99/50433 and US 6,204,006 and the artisan will be capable of preparing derivatives by these and other methods. Various routes for obtaining sphingosines are described by D. Shapiro in "Chemistry of Sphingolipids", Hermann, Paris (1969). Methods for producing certain phytosphingolipid derivatives are known to the skilled person, for instance it is known from US 6,204,006 and US 5,618,706 to derive tetraacetyl-phytosphingosine (TAPS) from microbial sources (i.e. *Pichia ciferrii*) and to subject this TAPS to hydrolysis to yield phytosphingosine.

A sphingolipid according to the formula (I), or a precursor, a derivative or a pharmaceutically acceptable salt thereof, may also be synthesized by known methods such as e.g. known from U.S. patents 5.232.837 and 5.110.987, or by standard modifications of these methods.

A known issue relating to the administration of sphingolipids, be it in foods or in pharmaceutical compositions, is that they can be metabolized. This is particularly relevant for application of sphingolipids in the digestive tract. This issue may be addressed by administering a sphingolipid according to the formula (I), more preferably according to formula (II) or (III) or (IV), or a derivative or a pharmaceutically acceptable salt thereof, alone or in combination, as a so-called precursor compound which compound comprises certain substituents as a result of which the compound can no longer, or only at reduced rates, be metabolized. These precursors are preferably resistant to hydrolysis in the upper parts of the digestive tract (e.g. mouth, stomach), and are for instance split relatively easy in the lower part of the digestive tract (e.g. coecum, colon), if the sphingolipid should have its working especially there. Preferably, when the intake of the precursor is via the oral route, the intact or metabolized precursors are taken up into the blood stream and transported to the target organs, especially liver, muscle and adipose tissue where they may be activated in order to exert their beneficial effect. Thus, it is possible that activation occurs when the compound has been absorbed from the digestive tract, e.g. in the blood or the liver. As a result, the amount of the compound is raised at those locations where the sphingolipid has its action. For instance, a sphingolipid precursor may be used that can be split or activated *in vivo* by a suitable enzyme so that the sphingolipid is liberated that may reduce the levels of cholesterol and triglycerides in the subject. Sphingolipid precursors have been described in WO 99/41266.

It is possible to modify a precursor of a sphingolipid according to the formula (I), (II), (III) or (IV) by an in situ enzymatic or chemical conversion, i.e. in the body, to a sphingolipid according to the formula (I), (II), (III) or (IV), which can be used in embodiments of the present invention. Such precursors of a sphingolipid according to the formula (I), (II), (III) or (IV) are therefore also suited for use according to the invention. A condition is that the precursor is converted in the body, e.g. preferably in the intestine, to a sphingolipid according to the formula (I), (II), (III) or (IV), e.g. by enzymatic conversion, in which case there is *in situ* activation. It is therefore, for instance possible to administer together with e.g. sphingomyelin, the enzyme sphingomyelin deacylase which may convert the sphingomyelin to lyso-sphingomyelin. Another possibility is to use sphingomyelinase to convert sphingomyelin into ceramide. In its turn ceramide can be broken down by ceramidase into a sphingoid base structure and a fatty acid. Other examples of enzymes may for instance be found in Sueyoshi *et al.,* (Sueyoshi N, Izu H, Ito M. 1997. J Lipid Res. 38(9):1923-7). Preferably, however, the sphingolipid according to the formula (I), (II), (III) or (IV) is not used as a precursor but in its "active" form in a combined pharmaceutical preparation according to the invention.

A combined preparation according to the invention comprising a sphingolipid and a HMG CoA reductase inhibitor, may be provided to a subject in need thereof for prophylactic or therapeutic reasons.

The combined preparation may be administered in any suitable pharmaceutical form. The pharmaceutical composition may be in the form of a capsule, tablet, lozenge, dragee, pill, droplet, suppository, powder, spray, vaccine, ointment, paste, cream, inhalant, patch, aerosol, and the like and may comprise a suitable pharmaceutically acceptable carrier for administration of the therapeutic agent.

As pharmaceutically acceptable carrier, any solvent, diluent or other liquid vehicle, dispersion or suspension aid, surface active agent, isotonic agent, thickening or emulsifying agent, preservative, encapsulating agent, solid binder or lubricant can be used which is most suited for a particular dosage form and which is compatible with the sphingolipid and/or the HMG CoA reductase inhibitor. Alternatively, suitable carriers may be large, slowly metabolized macromolecules such as proteins, polysaccharides, polylactic acids, polyglycolic acids, polymeric amino acids, amino acid copolymers, and inactive virus particles. Such carriers are well known to those of ordinary skill in the art. Preferred pharmaceutically acceptable carriers in therapeutic compositions are liquids such as water, saline, glycerol and ethanol. Additionally, auxiliary substances, such as wetting or emulsifying agents, pH buffering substances, and the like, may be present in such vehicles. The therapeutic compositions may also be prepared as injectables, either as liquid solutions or suspensions. Solid forms suitable for solution in, or suspension in, liquid vehicles prior to injection may also be prepared. Liposomes are included within the definition of a pharmaceutically acceptable carrier.

Pharmaceutically acceptable salts can also be used therein, for example, mineral acid salts such as hydrochlorides, hydrobromides, phosphates, sulfates, and calcium salts and the like; and the salts of organic acids such as acetates, propionates, malonates, benzoates, and the like. A thorough discussion of pharmaceutically acceptable excipients is available in Remington's Pharmaceutical Sciences (Mack Pub. Co., N.J. 1991).

For therapeutic treatment, the HMG CoA reductase inhibitor and sphingolipid may be produced as described in any of the references and descriptions provided above and applied to the subject in need thereof. The preparations may be administered to a subject by any suitable route, preferably in the form of a pharmaceutical composition adapted to such a route and in a dosage that is effective for the intended treatment. Therapeutically effective dosages of the sphingolipid required for treating the disorder, for instance for prevention and/or treatment of a disorder selected from the group consisting of hepatic steatosis, fibrosis and/or cirrhosis in the body of a human or animal subject, can easily be determined by the skilled person, for instance by using animal models.

For purposes of the present invention, an effective daily dosage of sphingolipid will be from about 0.01 mg/kg to 1 about g/kg, preferably from about 0.1 mg/kg to about 100 mg/kg, and most preferably from about 1 mg/kg to about 50 mg of the sphingolipid /kg body weight in the individual to which it is administered.

For purposes of the present invention, an effective daily dosage of a HMG CoA reductase inhibitor will be from about 0.001 mg/kg to about 10 mg/kg, preferably from about 0.01 mg/kg to about 1 mg/kg and most preferably about 0.1 mg/kg to about 0.25 mg of a HMG CoA reductase inhibitor /kg body weight in the individual to which it is administered.

Dosages for achieving the therapeutic effects of the preparations described herein may easily be determined by the skilled person

Preferably a pharmaceutical composition as described above is intended for oral application, and although intravenous or intramuscular administration is also possible, oral administration is preferred. Compositions for oral application will usually comprise an inert diluent or an edible carrier. The compositions may be packed in e.g. gelatin capsules or may be tabletted in the form of tablets. For oral therapeutic application the active compound may be administered with excipients and e.g. used in the form of powders, sachets, tablets, pills, pastilles or capsules. Pharmaceutically acceptable binders and/or adjuvants may also be comprised as constituents of the pharmaceutical composition.

The powders, sachets, tablets, pills, pastilles, capsules and such may comprise each of the following components or compounds of similar import: a filler such as microcrystalline cellulose (MCC) or mannitol; a binder such as hydroxypropylcellulose (HPC), tragacanth gum or gelatin; an excipient such as starch or lactose; a desintegrant such as alginate or corn starch; a lubricant such as magnesium stearate; a sweetener such as sucrose or saccharose; or a flavoring substance such as peppermint or methyl salicylic acid.

When dosing is in the form of a capsule, the capsule may comprise apart from the elements mentioned above a liquid carrier such as an oil. Dosage form may further be provided with coatings of sugar, shellac or other agents. The components of the pharmaceutical composition are preferably chosen such that they do not reduce the desired working of the sphingolipid.

In a pharmaceutical composition as described above, a sphingolipid is used in an amount of from 0.01 to 99.9 % by (dry) weight, preferably from 0.1 to 50 wt.%, and more preferably from 1 to 10 wt.%. Preferably, the pharmaceutical composition is in the form of an oral dosage form with which an amount of 0.1-10 grams of sphingolipid can be consumed in a single dosage.

In a pharmaceutical composition as described above, a HMG CoA reductase inhibitor is used in an amount of from 0.01 to 99.9 % by (dry) weight, preferably from 0.1 to 50 wt.%, and more preferably from 1 to 10 wt.%.

In a combined preparation for simultaneous administrations, a sphingolipid may be present in an amount of from 0.01 to 99.9 % by (dry) weight, preferably from 0.1 to 50 wt.%, and more preferably from 1 to 10 wt.%, and a HMG CoA reductase inhibitor may be present in an amount of from 99.9 to 0.01 % by (dry) weight, preferably from 50 to 0.1 wt.%, and more preferably from 10 to 1 wt.%. Suitable amounts of sphingolipids and HMG CoA reductase inhibitor in a combined preparation of the present invention, are from about 0.5 mg to 50 g, preferably from about 5 mg to about 5 g, and most preferably from about 50 mg to about 2.5 g of the sphingolipid, and from about 0.05 mg to about 500 mg, preferably from about 0.5 mg to about 50 mg and most preferably about 5 mg to about 20 mg of a HMG CoA reductase inhibitor per unit dosage form.

A pharmaceutical composition according to the invention is intended for treating or preventing hypercholesterolemia, while simultaneous treating or preventing liver damage in a subject.

Although the individual cholesterol lowering activity of both sphingolipids and the HMG CoA reductase inhibitor were known, a synergistic effect was hitherto not described. The cholesterol and triglycerides in blood are lowered more effectively than when both compounds are used alone. Moreover, the rise in ALT and SAA values caused by statins can be prevented by sphingolipids, as a result of which the dosage of the HMG CoA reductase inhibitor can be raised, or the same level reduction can be attained by using lower dosages.

The present invention further relates to a method for the preparation of a pharmaceutical composition for the prevention and/or treatment of a disorder selected from the group consisting of hepatotoxic effects of compounds such as alcohol and medicaments, hepatic steatosis, fibrosis and/or cirrhosis in a subject, comprising processing or incorporating a sphingolipid according to the formula (I), (II), (III) or (IV), or a precursor, a derivative or a pharmaceutically acceptable salt thereof, as an active substance, together with a pharmaceutically acceptable carrier in a pharmaceutical composition.

The preparation of a pharmaceutical composition may very suitably occur by compounding or admixing sphingolipid and a HMG CoA reductase inhibitor, optionally in combination with additional ingredients such as fillers, binders, lubricants and other excipients, and forming the mixture obtained to a pharmaceutical preparation, for instance by tabletting.

### EXAMPLE

### Introduction

In has been shown that a milk lipid preparation enriched in sphingomyelin is capable of reducing plasma (VLDL and LDL) cholesterol and triglyceride levels in hypercholesterolemic APOE*3Leiden mice (see WO 2004/064820). In humans, this effect was demonstrated decisively for phytosphingosine. The pharmaceutical compound Atorvastatin (a statin, marketed as Lipitor®) is a potent plasma cholesterol lowering drug by inhibiting the HMG CoA-reductase, and thus the synthesis of cholesterol. Combined administration of sphingolipids and statins reveals a synergistic effect on the reduction of plasma cholesterol between these compounds.

The combined effect of milk sphingomyelin (mSM) and statin on plasma levels of cholesterol and triglycerides is demonstrated by providing these compounds alone and in combination to female APOE*3Leiden mice fed a hypercholesterolemic diet. Phytosphingosine is used as a positive control.

### Materials and Methods

APOE*3Leiden mice are transgenic mice, carrying the human APOE*3Leiden gene. In mice, the human mutant apolipoprotein E3-Leiden dominantly inhibits hepatic uptake of VLDL and IDL (which is a very efficient process in wild-type mice). Plasma cholesterol levels in these mice are exquisitely sensitive to the amount of cholesterol in the diet; hence, the plasma cholesterol level can be titrated to any desired level by changing the dietary cholesterol content. A large number of studies have shown that in APOE*3Leiden mice plasma cholesterol levels are amenable to pharmacological and nutritional modification.

### Animals

Three months-old female heterozygous E3L mice from the SPF breeding stock at the TNO Gaubius Building (Leiden) are used, and housed during the experiment in clean-conventional animal rooms at TNO Leiden (relative humidity 50-60%, temperature ~21°C, light cycle 6 am to 6 pm). Mice are housed in macrolon cages (five mice per cage) and are weighed every week. Food consumption is determined weekly on a cage basis. Blood (for EDTA plasma) is obtained from a tail vein (no anaesthesia).

### Sacrifice

At sacrifice, after the tail blood sample has been obtained, the mice are euthanized by cervical dislocation. The liver is weighed, snap-frozen, and stored below -60°C for possible later analysis. Jejunum and ileum are dissected and stored frozen at -60°C for possible later analysis.

### Materials

Milk sphingomyelin (mSM) will be prepared using a similar procedure as described by Thompson and Singh (J. Dairy Sci. 89, 410-419 (2006). Yeast phytosphingosine is obtained from Cosmoferm (Delft, the Netherlands).

### Diets

Dietary ingredients are provided by Hope Farms (Woerden, the Netherlands). The semi-synthetic high cholesterol (HC) diet (Western-type diet) will contain 40.5% sucrose, 20% casein, 15% cocoa butter, 10% corn starch, 5.45% cellulose, 5.1% mineral mixture, 1% choline chloride, 1% corn oil, 0.2% methionine, and 0.2% cholesterol (all w/w). Food and water are given *ad libitum.* Diets are prepared by stepwise dilution of compound(s) with the powdered diet, mixing with 2% agar, and freeze-drying as pellets. The diets are prepared once, and stored in the dark at -20°C.

### Experimental setup

A total of 80 heterozygous APOE*3Leiden (E3L) mice are provided. The mice are fed a high-cholesterol diet for four weeks (week 1 to 4), establishing plasma cholesterol levels of around 12 mmol/L in those mice. At week 0, the mice are randomized on the basis of plasma total cholesterol level into a control group and 6 treatment groups (ten mice each).

The twelve groups are:
1. Hypercholesterolemic diet (HC diet) (control group)
2. HC diet plus phytosphingosine (0.1%) (positive control group)
3. HC diet plus milk sphingolipid preparation (mSM 0.1% w/w)
4. HC diet plus Atorvastatin (25 mg/kg diet)
5. HC diet plus Atorvastatin (25 mg/kg diet) plus milk sphingolipid preparation (mSM 0.1% w/w)
6. HC diet plus Atorvastatin (25 mg/kg diet) plus milk sphingolipid preparation (mSM 0.2% w/w)
7. HC diet plus Atorvastatin (25 mg/kg diet) plus milk sphingolipid preparation (mSM 0.4% w/w)

Selected dosages of milk sphingolipids are aimed at a reduction of plasma cholesterol by 10% by milk sphingolipids alone, and are established on the basis of pilot studies. The concentration of Atorvastatin is established based on the previously observed effects of statins in APOE*3Lleiden mice, and aims at a reduction of plasma cholesterol by 20%.

From week 5, groups are treated with the above-mentioned compound(s) in the diet.

### Analysis

Plasma total cholesterol and total triglycerides are determined in all animals individually, and lipoprotein distribution (fplc analysis) and ALAT are determined at group level.
Body weight is determined every two weeks, food intake weekly in week 5-8.
EDTA plasma (tail blood) is obtained (after a four-hour fast) for lipid and
ALAT analysis at week 4, 5, 6, and 8.
At autopsy, livers are weighed and snap-frozen.

### Lipid analysis

In EDTA plasma from tail blood is determined, for each mouse individually, at weeks 5, 6, and 8
■ total cholesterol (kit from Roche, Mannheim)
■ total triglycerides (kit from Sigma, MI)
Lipoprotein distribution is determined, group-wise, by fast protein liquid chromatography (AKTA fast protein liquid chromatography system;
Amersham-Pharmacia Biotech, UK) in week 4, 6 and 8.

### ALAT

ALAT (spectrophotometric assay, Boehringer Reflotron system) is determined at sacrifice individually in all mice, and group-wise in week 4 and 6.
In case of an abnormal body weight development and/or food consumption, ALAT is determined individually at the next sampling point in the mouse (mice) involved.

## Claims

1. Use of a sphingolipid and a cholesterol lowering agent, wherein said cholesterol lowering agent is a HMG-CoA reductase inhibitor, for the manufacture of a combined preparation for simultaneous, separate or sequential use in the treatment of hypercholesterolemia in a subject in need thereof.

2. Use according to claim 1, wherein said treatment of hypercholesterolemia involves the simultaneous treatment or prophylaxis of liver damage.

3. Use according to claim 1 or 2, wherein said cholesterol lowering agent is administered to said subject prior to or following the administration of the sphingolipid.

4. Use according to any one of claims 1-3, wherein said sphingolipid has the general formula (I): 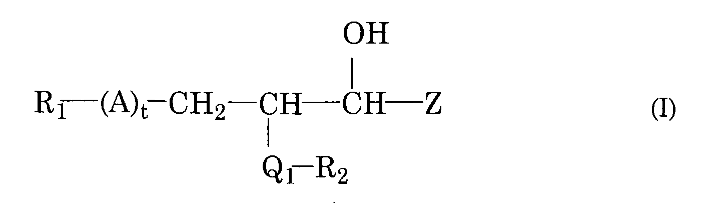 wherein
Z is R₃ or -CH(OH)-R₃;
A is sulphate, sulphonate, phosphate, phosphonate or -C(O)O-;
R₁ is H, hydroxyl, alditol, glycosyl, an alcohol, C₁-C₆ alkyl or amino acid;
R₂ is H or unsaturated or saturated (C₁-C₃₀) alkyl chain;
R₃ is unsaturated or saturated (C₁-C₃₀) alkyl chain;
Q₁ is a primary amine group (-NH₂), secondary amine group (-NH-) or an amide group (-NH-CO-).

5. Use according to any one of claims 1-3, wherein said sphingolipid has the general formula (II) 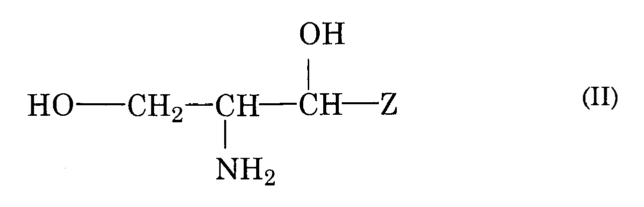 wherein
Z is R₃ or CH(OH)-R₃, and
R₃ is an unsaturated or saturated (C₁-C₃₀) alkyl chain.

6. Use according to any one of claims 1-3, wherein said sphingolipid has the general formula (III) 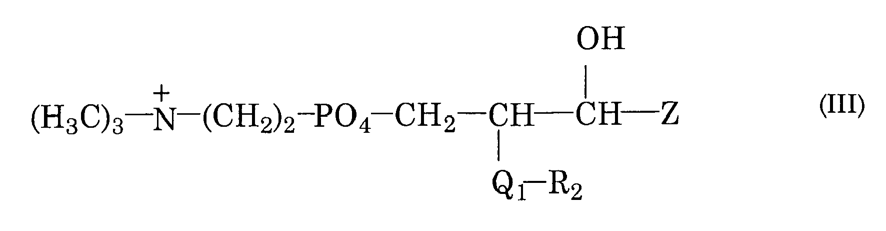 wherein
Z is R₃ or CH(OH)-R₃, preferably R₃;
Q₁ is a primary amine group (-NH₂), a secondary amine-group (-NH-) or an amide group (-NH-CO-); preferably an amide group, and
R₂ is H or unsaturated or saturated (C₁-C₃₀) alkyl chain;
R₃ is an unsaturated or saturated (C₁-C₃₀) alkyl chain, preferably an unsaturated (C₁-C₃₀) alkyl chain.

7. Use according to any one of claims 1-3, wherein said sphingolipid has the general formula (IV), 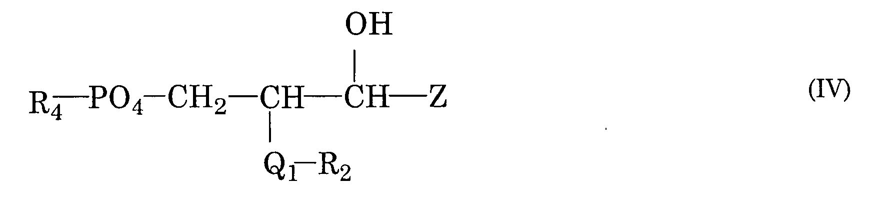 wherein
Z, Q₁, and R₂ are as defined above, and
R₄ is selected from H, hydroxyl, alditol, glycosyl, alcohol, C₁-C₆ alkyl or amino acid, provided that when R₄ is H, Q1-R₂ is not the primary amine group. The hydroxyl, alditol, alcohol, C₁-C₆ alkyl or amino acid can for insntance be those compounds as named for R₁ above. In case R₄ is glycosyl, it may be selected from the group of radicals consisting of acesulfam, allose, altrose, arabinose, erythrose, fructose, fucose, galactose, glucose, gulose, idose, isomaltose, lactose, lyxose, maltose, mannose, melezitose, psicose, raffinose, rhamnose, ribose, saccharose, sorbose, stachyose, sucrose, tagatose, talose, threose, trehalose, turanose, xylose and xylulose, and other mono-, di-, or polysaccharides.

8. Use according to any one of claims 1-3, wherein said sphingolipid is selected from the group consisting of phytosphingosine, sphingosine, sphinganine, ceramide, glycosylceramide, sphingomyelin, and derivatives thereof, and combinations thereof, preferably said sphingolipid is phytosphingosine.

9. Use according to any one of the preceding claims, wherein said cholesterol lowering agent is a statin, preferably a statin selected from the group consisting of atorvastatin, cerivastatin, fluvastatin, lovastatin, mevastatin, pitavastatin, pravastatin, rosuvastatin, and simvastatin.

10. Use according to any of the preceding claims, wherein the daily dose of the sphingolipid is between about 0.1 mg/kg and about 50 mg/kg body weight.

11. Use according to claim 9 or 10, wherein the statin is administered at a dose of about 0.001 to about 5 mg/kg body weight.

12. A combined preparation of a sphingolipid and at least one additional cholesterol lowering agent, wherein said additional cholesterol lowering agent is a HMG-CoA reductase inhibitor.

13. A combined preparation of a sphingolipid and at least one additional cholesterol lowering agent, wherein said additional cholesterol lowering agent is a HMG-CoA reductase inhibitor, for the simultaneous, separate or sequential use in therapy.

14. A combined preparation according to claim 12 or 13, wherein said sphingolipid is selected from the group consisting of phytosphingosine, sphingosine, sphinganine, ceramide, glycosylceramide, sphingomyelin, and derivatives thereof, and combinations thereof.

15. A combined preparation according to any one of claims 12-14, wherein said cholesterol lowering agent is a statin.

16. Method of treating hypercholesterolemia in a subject, said method comprising administering to said subject a therapeutically effective amount of a preparation according any one of claims 12-15.
